# EUROPÄISCHE PATENTANMELDUNG

(11) **EP 1 803 660 A1**
(43) Veröffentlichungstag der Anmeldung: **04.07.2007**
(21) Anmeldenummer: 06025395.2
(22) Anmeldetag: 08.12.2006
(51) Int. Cl.: B65D 83/14, A61K 8/06

(54) **Emulsionsspray**

(30) Priorität: 28.12.2005 DE 102005062960
(71) Anmelder: Henkel Kommanditgesellschaft auf Aktien, 40589 Düsseldorf (DE)
(72) Erfinder: Emmerling, Winfried, 25436 Tornesch (DE); Bergemann, Uwe, 22459 Hamburg (DE); Kaftan, Pamela, 25451 Quickborn (DE); Heinsohn, Ulrike, 22529 Hamburg (DE)
(74) Vertreter: Augustin-Castro, Barbara

(57) **Zusammenfassung**

Gegenstand der vorliegenden Erfindung ist ein kosmetisches Mittel, insbesondere ein schweißhemmendes und/oder deodorierendes Mittel, in Form einer mindestens einen kosmetischen Wirkstoff enthaltenden Wasser-in-Öl-Emulsion, die in einer Abgabevorrichtung zum Versprühen als Aerosol mit einer niedrigen Sprührate verpackt ist.

## Beschreibung

Die vorliegende Anmeldung betrifft ein kosmetisches Mittel, insbesondere ein schweißhemmendes und/oder deodorierendes Mittel, in Form einer mindestens einen kosmetischen Wirkstoff enthaltenden Wasser-in-Öl-Emulsion, die in einer Abgabevorrichtung zum Versprühen als Aerosol mit einer niedrigen Sprührate verpackt ist.

Ein Aerosol ist ein disperses System, bei dem ein Feststoff oder eine Flüssigkeit in einem Gas sehr fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel- Zubereitungsgemisch durch eine feine Düse, das Treibmittel verdunstet und hinterlässt das fein verteilte Sprühgut als Aerosol.
Aerosol-Antitranspirantsprays erfreuen sich im Körperpflegebereich zunehmender Beliebtheit. Gängige Antitranspirantsprayzusammensetzungen liegen als wasserfreie Suspensionen des pulverförmigen schweißreduzierenden Wirkstoffes, meist eines Aluminiumsalzes, neben dem Treibgas in einem flüssigen Träger, meist einem relativ flüchtigen Öl wie Cyclomethicone, vor. Zur besseren Suspendierung des pulverförmigen Wirkstoffes enthält der flüssige Träger häufig noch ein Verdickungsmittel, zum Beispiel Bentone-Gel. Vor dem Versprühen muss die Suspension aufgeschüttelt werden. Ein Nachteil dieser Suspensionsaerosole ist das Risiko, dass die Ventil- bzw. Düsenbohrungen bei höheren Einsatzkonzentrationen des Salzes verstopfen. Es gab daher Versuche, das Antitranspirant-Salz in gelöster Form zu versprühen. Jedoch verursacht die Konfektionierung wässriger Antitranspirant-Salzlösungen in Treibmittel-haltigen Metalldosen große Korrosionsprobleme bei der Aerosolverpackung, so dass selbst bei lackierten Spraydosen unweigerlich Korrosionserscheinungen an der Dose auftreten.
Gängige Deodorantsprayzusammensetzungen liegen als wasserfreie ethanolische Lösungen vor. Nachteilig ist, dass die Einarbeitung wasserhaltiger oder wasserlöslicher Deodorant-Wirkstoffe, die nicht auch in Ethanol löslich sind, nicht oder nur sehr eingeschränkt möglich ist. Auch hier führte der Zusatz selbst geringer Mengen Wasser zu Korrosionserscheinungen an den Standardventilen und sogar an lackierten Spraydosen.
Es gab weiterhin Versuche, diese Korrosionsrisiken durch den Einsatz von Wasser-in-Öl-Emulsionen, bei denen der Antitranspirant- und/oder Deodorant-Wirkstoff in der inneren wässrigen Phase gelöst ist, zu vermindern (WO 2004/030641 A1, WO 96/24326 A1). Dabei erhoffte man, dass die äußere Ölphase den Kontakt der inneren, korrosiv wirkenden wässrigen Phase mit der Dose und dem Ventil verhindert. Trotz intensiver Bemühungen war es aber bislang nicht möglich, vollständig befriedigende Produkte zu entwickeln. Die Korrosion konnte nur kurzfristig unterdrückt werden, doch stellt dies keine Lösung für den Bereich der Konsumentenprodukte dar, bei denen von der Herstellung bis zur Verwendung der letzten Produktreste mitunter mehrere Jahre vergehen können. Hier muss für den Kunden sichergestellt sein, dass Produkt und Applikationssystem keiner Veränderung wie Korrosion unterliegen und auch nach dieser Zeit noch einwandfrei funktionieren. Eigene Versuche haben gezeigt, dass auch mit Formulierungen auf Basis einer Antitranspirant- und/oder Deodorant-Wirkstoffe enthaltenden Wasser-in-Öl-Emulsion Korrosion an den eingesetzten Standardventilen, insbesondere an den metallischen Ventilfedern, zu beobachten ist.

Kosmetische Zusammensetzungen, insbesondere Deodorantsprays und Antitranspirantsprays, werden durchaus mehrfach am Tag angewendet. Dabei wird das entsprechende kosmetische Mittel häufig vom Verbraucher griffbereit mitgeführt. Dabei ist jedoch das große Volumen der gebräuchlichen Aerosoldosen ein großer Nachteil. Zwar könnten die Aerosoldosen im Volumen reduziert werden, beispielsweise auf Dosen von 100 ml oder 50 ml Inhalt, dann würde der Inhalt jedoch nur für wenige Anwendungen ausreichen. Dies ist für den Verbraucher nicht akzeptabel und erhöht zudem das Abfallaufkommen. Abhilfe könnten hier hochkonzentrierte, mindestens doppelt bis fünffach so hoch konzentrierte auf den Gehalt an kosmetischem Wirkstoff, insbesondere an Deodorant- und/oder Antitranspirant-Wirkstoff bezogene kosmetische Rezepturen schaffen.

Aus der EP 674 899 A2 sind Deodorants in Form von Aerosolen bekannt, die aus ökologischen Gründen in möglichst kleine Gebinde verpackt werden. Um dabei die Sprührate bei einem aufkonzentrierten Deospray so zu erhalten, dass nicht zuviel Produkt ausgetragen wird, wurden die Formulierungen mit Hydroxypropylcellulose verdickt. Bei den offenbarten Rezepturen handelt es sich um einphasige Lösungen des deodorierenden Wirkstoffes in einem Lösemittel, bevorzugt kurzkettige einwertige Alkohole wie Ethanol, oder um flüchtige Silicone, wobei bevorzugt nicht mehr als 10 Gew.-% Wasser enthalten sind. Dieser Druckschrift war also weder ein Hinweis auf die Formulierung kompakter W/O-Emulsionssprays noch auf die damit verbundene Korrosionsproblematik zu entnehmen.
Antitranspirant-Sprayzusammensetzungen auf Basis von siliconölhaltigen Wasser-in-Öl- bzw. Wasser-in-Siliconöl-Emulsionen sind bekannt. WO 96/24326 A1 beschreibt eine Zubereitung mit 10 bis 50 % einer W/O-Emulsion, die ein Aluminiumsalz enthält, und 50 bis 90 % eines Treibgases, wobei die Zubereitung in einer Aluminiumdose verpackt sein kann. Das Problem der korrosiven Wirkung solcher Zusammensetzungen auf die Bestandteile der Verpackung und der Abgabevorrichtung wird in dieser Offenlegungsschrift nicht angesprochen.
Die WO 94/22420 A1 beschreibt Aerosole auf Basis von silikonhaltigen Wasser-in-Öl-Mikroemulsionen, die nach dem Verdampfen des Treibgases auf der Haut klare Gele bilden. Auch diese Schrift beschäftigt sich nicht mit dem bestehenden Korrosionsproblem. Kosmetische Sprayzusammensetzungen auf Basis von siliconölhaltigen Wasser-in-Öl- bzw. Wasser-in-Siliconöl-Emulsionen, die feuchtigkeitsspendende und andere hautpflegende Wirkstoffe enthalten, sind ebenfalls bekannt, beispielsweise aus WO 2004/030641 A1. Diese Druckschriften offenbaren jedoch nicht, dass diese Zusammensetzungen auch mit niedriger Sprührate ausgestoßen werden können. Sprays mit niedriger Sprührate herzustellen, ist für den Fachmann nicht trivial. Die üblicherweise verwendeten Formeln müssen überarbeitet werden, da eine deutlichen Erhöhung des gewichtsbezogenen Emulsions-/Treibgas-Verhältnisses häufig zu einer Verstopfung des Ventils führt. Außerdem muss im Gegensatz zu Suspensionssprays, bei denen der kosmetische Wirkstoff, beispielsweise ein festes Antitranspirantsalz, in ungelöster Form suspendiert vorliegt, oder zu Lösungssprays, bei denen der kosmetische Wirkstoff in gelöster Form in einer einphasigen Lösung vorliegt, bei der Formulierung von Emulsionen die Emulsionsstabilität berücksichtigt werden. Da die meisten kosmetischen Wirkstoffe wasserlöslich und seltener öllöslich sind, wie das insbesondere auf Deodorant- und/oder Antitranspirant-Wirkstoffe zutrifft, kann die Konzentrationserhöhung in der inneren wässrigen Emulsionsphase einer Wasser-in-Öl-Emulsion zum vorzeitigen Brechen der Emulsion führen. Dies hätte aber für eine treibgaskomprimierte Zusammensetzung in einem metallischen Aerosolbehälter verheerende Konsequenzen in Bezug auf die Korrosion des Behälters und ist daher unbedingt zu vermeiden.

Eine Aufgabe der vorliegenden Erfindung war es, ein kosmetisches oder dermatologisches Produkt auf Basis einer wasserhaltigen Emulsion mit mindestens einem kosmetischen oder dermatologischen Wirkstoff mit einer Abgabevorrichtung zu entwickeln, das eine verbesserte Lagerstabilität aufweist. Eine weitere Aufgabe war es, ein kosmetisches oder dermatologisches Produkt auf Basis einer wasserhaltigen Emulsion mit mindestens einem kosmetischen oder dermatologischen Wirkstoff mit einer Abgabevorrichtung zu entwickeln, das verringerte Korrosionseigenschaften aufweist.
Eine weitere Aufgabe der vorliegenden Erfindung war es, ein Antitranspirant- und/oder Deodorant-Produkt auf Basis einer wasserhaltigen Emulsion mit Antitranspirant- und/ oder Deodorant-Wirkstoffen mit einer Abgabevorrichtung zu entwickeln, das eine verbesserte Lagerstabilität aufweist. Eine weitere Aufgabe war es, ein Antitranspirant- und/oder Deodorant-Produkt auf Basis einer wasserhaltigen Emulsion mit Antitranspirant- und/ oder Deodorant-Wirkstoffen mit einer Abgabevorrichtung zu entwickeln, das verringerte Korrosionseigenschaften aufweist.
Weiterhin bestand die Aufgabe, entsprechende hochkonzentrierte Mittel zu entwickeln, die hinsichtlich der anwendungstechnischen Eigenschaften, beispielsweise einer lang andauernden schweißhemmenden und/oder deodorierenden Wirkung, die Erwartungen der Verbraucher erfüllen. Zusätzlich wird vom Verbraucher eine kleine Verpackung, die sich bequem transportieren lässt und somit überall verfügbar ist, gewünscht. Trotz der gewünschten kleinen Verpackung soll jedoch der Inhalt für zahlreiche Anwendungen ausreichen und der Zahl der Anwendungen eines handelsüblichen Sprays, insbesondere eines Deodorant- oder Antitranspirant-Sprays, entsprechen oder diese im besten Fall sogar übertreffen.

Überraschend wurde nun gefunden, dass die bestehenden Korrosionsprobleme sowie die übrigen bestehenden Probleme konzentrierter Emulsionssprays überwunden werden können. Von entscheidender Bedeutung ist dabei die Wahl der verwendeten Materialien für das Ventil der Abgabevorrichtung sowie die erfindungsgemäße Auswahl der Komponenten der Emulsion. Erfindungsgemäß geeignete Ventile zeichnen sich dadurch aus, dass sie keine Federn oder flexiblen Elemente mit Rückstellcharakteristik enthalten, deren Kontaktfläche mit der kosmetischen Zusammensetzung aus metallischen Materialien besteht oder aber dadurch, dass sie überhaupt keine Feder enthalten.

Ein Gegenstand der vorliegenden Erfindung ist daher ein kosmetisches Produkt, das eine Wasser-in-Öl-Emulsion mit mindestens einem kosmetischen oder dermatologischen Wirkstoff, mindestens ein Treibmittel und eine Aerosol-Abgabevorrichtung umfasst, wobei die mit der Emulsion in Kontakt kommenden Teile des Ventils der Abgabevorrichtung aus nicht-metallischen Materialien bestehen und die Emulsion mit einer Sprührate von 0,05 - 0,5 g/s, bevorzugt 0,1 - 0,35 g/s, besonders bevorzugt 0,15 - 0,2 g/s, ausgestoßen wird.

In einer ersten bevorzugten Ausführungsform der Erfindung weist das Ventil einen mit einem Lack oder einem polymeren Kunststoff A beschichteten Ventilkegel und ein ebensolches flexibles Element mit Rückstellcharakteristik auf, welches derart angeordnet ist, dass das Ventil nach Beenden der Betätigung in die Verschlussstellung (= Ruhelage des Ventils) zurückgestellt wird. In einer weiteren bevorzugten Ausführungsform der Erfindung weist das Ventil ein flexibles Element mit Rückstellcharakteristik und/oder einen Ventilkegel aus mindestens einem Kunststoff B, bevorzugt einem elastomeren Kunststoff, auf. Bevorzugte elastomere Kunststoffe sind ausgewählt aus Buna, insbesondere Buna N, Buna 421, Buna 1602 und Buna KA 6712, Neopren, Butyl und Chlorbutyl. In einer weiteren bevorzugten Ausführungsform der Erfindung kann das flexible Element mit Rückstellcharakteristik als Spiralfeder bzw. Schraubendruckfeder ausgebildet sein. In einer weiteren bevorzugten Ausführungsform der Erfindung kann das flexible Element mit Rückstellcharakteristik einstückig mit dem Ventilkegel ausgebildet sein und biegsame Beine aufweisen. In einer besonders bevorzugten Ausführungsform der Erfindung sind Ventilkegel und flexibles Element mit Rückstellcharakteristik ähnlich, äquivalent oder identisch wie in WO 89/08062 A1, Figur 1 und den dazugehörigen Erläuterungen dargestellt, ausgebildet. Besonders bevorzugt ist dabei der Ventiltyp Ariane M, erhältlich von der Firma Seaquist Perfect, bei dem das flexible Element mit Rückstellcharakteristik in Form von vier elastischen Beinen einstückig mit dem Ventilkegel ausgebildet ist.
Erfindungsgemäß bevorzugt ist auch eine Ventilkonstruktion gemäß US 4,471,893.
In einer weiteren bevorzugten Ausführungsform der Erfindung weist die Abgabevorrichtung ein federloses Ventil auf, wie es zum Beispiel Gegenstand der US 2003/0102328 A1 ist.

Alle erfindungsgemäß verwendeten Ventile weisen einen innenlackierten Ventilteller auf, wobei Lackierung und Ventilmaterial miteinander kompatibel sind. Werden erfindungsgemäß Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Die eingesetzten Behälter, die z. B. aus Weißblech oder aus Aluminium sein können, wobei Aluminiumbehälter erfindungsgemäß bevorzugt sind, müssen angesichts der Korrosivität der erfindungsgemäß verwendeten W/O-Emulsionen ebenfalls innen lackiert oder beschichtet sein. Ein erfindungsgemäß bevorzugter Innenschutzlack ist ein Epoxy-Phenollack, wie er u.a. unter der Bezeichnung Hoba 7407 P erhältlich ist.

Die Dosen sind mit einem geeigneten Sprühkopf ausgestattet. Je nach Sprühkopf sind Ausstoßraten, bezogen auf voll gefüllte Dosen, von 0,05 g/s bis 0,5 g/s möglich. Die Sprührate wird dabei so bestimmt, dass eine mit Treibgas und der entsprechenden Zusammensetzung gefüllte und mit dem betreffenden Ventil verschlossene Aerosoldose unter Normalbedingungen (23 °C) zunächst gewogen wird, außerdem wird ihr Anfangsinnendruck in Anlehnung an den FEA Standard 643 E bestimmt. Die Dose wird samt Inhalt zehnmal kräftig von Hand geschüttelt, damit sich der Inhalt gut vermischt. Dann wird ein erster Sprühstoß von 5 Sekunden [s] Dauer versprüht und verworfen, um nicht-homogenes Material aus dem Tauchrohr zu entfernen. Dann wird für 10 s das Ventil der senkrecht stehenden Dose betätigt. Danach wird wiederum gewogen. Der Vorgang wird fünfmal hintereinander durchgeführt und der arithmetische Mittelwert aus den Ergebnissen gebildet. Die Differenz zweier aufeinanderfolgender Wägungen ist die Sprührate pro 10 s. Daraus lässt sich durch einfaches Dividieren die Sprührate je Sekunde bestimmen. Nach dem Sprühen wird jeweils der Innendruck der Dose bestimmt.

Ein weiterer charakteristischer Parameter für die Effizienz und Formulierbarkeit als Kompaktspray ist das Sprühbild. Das Sprühbild wird durch das Ventil und dessen Beschaffenheit entscheidend beeinflusst. Wenn beispielsweise in einer Kompaktsprayformulierung der kosmetische Wirkstoff, insbesondere der Deodorant- und/oder Antitranspirant-Wirkstoff, bis auf das Fünffache gegenüber einer konventionellen Rezeptur erhöht wird, so sind neben den zu beachtenden und zu vermeidenden erhöhten Viskositäten der Rezeptur auch die bereits diskutierte Sprührate wesentliche für die Formulierung zu beachtende Merkmale. Zusätzlich müssen jedoch insbesondere auch das Sprühbild, das heißt der Öffnungskegel des Ventils, und die Tröpfchengröße beachtet und geeignet ausgewählt werden.

Wenn der Öffnungskegel einen zu großen Öffnungswinkel aufweist, dann wird das Produkt bei einem üblichen Abstand der Sprühdose vom Körper des Anwenders von etwa 10 bis 40 cm auf eine zu kleine Körperoberfläche aufgebracht. Dies führt zu einer Veränderung der Wirksamkeit der Zusammensetzung. Es tritt entweder ein unangenehmes, klebriges oder belastetes Hautgefühl durch zu hohe Produktmengen oder eine zu geringe kosmetische, insbesondere deodorierende und/oder schweißhemmende Wirkung durch eine zu geringe aufgebrachte Produktmenge auf. im letzteren Falle ist der Öffnungskegel zu groß, so dass eine zu große Körperoberfläche mit der Zusammensetzung behandelt wird. Es hat sich nun gezeigt, dass der Öffnungskegel bevorzugt von 25° bis 65°, besonders bevorzugt von 30° bis 60° und außerordentlich bevorzugt von 35° bis 50° reicht.

Der Einfluss der Tröpfchengröße ist wie folgt: bei zu großen Tröpfchen ist die Verteilung der Rezeptur auf der zu behandelnden Körperoberfläche nicht gleichmäßig, so dass an vielen Stellen zu hohe Produktmengen auftreten. An anderen Stellen demgegenüber treten zu geringe Produktmengen auf. In zahlreichen Versuchen hat sich nun gezeigt, dass die zahlenmittlere Tröpfchengröße, bezogen auf den Tröpfchendurchmesser, bevorzugt weniger als 50 µm, besonders bevorzugt weniger als 45 µm und außerordentlich bevorzugt weniger als 40 µm, beträgt.

Die Tröpfchengröße wird dabei mit einem Laserbeugungsmessgerät vom Typ Masterizer, Series 2600 Droplet and Particle Size Analyzer der Firma Malvern bestimmt. Hierzu wird die Probe in einem definierten Abstand durch den Lichtstrahl des Lasers gesprüht und anhand der Laserbeugung die (zahlenmittlere) Teilchengrößenverteilung bestimmt.

Die Viskosität der zu versprühenden Rezeptur kann je nach Konzentration des kosmetischen Wirkstoffs ebenfalls einen Einfluss auf die Produktleistung und/oder die Sprührate zeigen. Es kann erfindungsgemäß bevorzugt sein, wenn die Viskositäten der Rezepturen kleiner als 5000 mPas, gemessen nach Brookfield mit Spindel 3 bei 20 Upm und 25 °C, betragen. Die Viskositäten der Rezepturen werden dabei vor der Zugabe von Treibgas gemessen. Besonders bevorzugt kann es sein, wenn die Viskositäten kleiner als 2500 mPas und ganz besonders bevorzugt kleiner als 1000 mPas sind.

Schließlich kann das Problem des Verklebens der Ventile zusätzlich zur gezielten Auswahl der kosmetischen Wirkstoffe, insbesondere der Antitranspirant- und/oder Deodorant-Wirkstoffe, auch durch eine sorgfältige Verarbeitung, Materialauswahl und/oder Vorbehandlung der Ventile positiv beeinflusst werden. Wesentlich ist dabei, dass alle mit der Zusammensetzung in Berührung kommenden Teile des Ventils eine möglichst glatte Oberfläche aufweisen. Je glatter die Oberfläche ist, desto weniger kann die Zusammensetzung an ihr durch Adhäsion anhaften. Somit wird einer Verklebung der Düse entgegengewirkt. Die Erzielung besonders glatter Oberflächen ist dem Fachmann bekannt, beispielsweise durch eine Aüsführung der Oberfläche in Form von Nanopartikeln zur Erzielung eines Lotusblüteneffektes oder einer Polierung der Oberflächen wie beispielsweise einer Elektropolierung.

Die Wasser-in-Öl-Emulsion des erfindungsgemäßen kosmetischen Produktes umfasst eine Ölphase, die bevorzugt 1 - 60 Gew.-%, besonders bevorzugt 10 - 50 Gew.-% und außerordentlich bevorzugt 15 - 35 Gew.-%, jeweils bezogen auf das Gesamtgewicht der Emulsion, ausmacht. Die Emulgatoren werden erfindungsgemäß weder zur Ölphase noch zur Wasserphase gezählt.
In einer weiteren bevorzugten Ausführungsform der Erfindung besteht die Ölphase zu mindestens 90 Gew.-% aus bei 20 °C flüssigen Ölkomponenten. Bevorzugte Ölkomponenten sind ausgewählt aus:
- flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind,
- nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5 - 100 cSt, bevorzugt 5 - 50 cSt oder auch 5 - 10 cSt, und Baysilon^{®} 350 M;
- den Estern von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen 2-Ethylhexylpalmitat (z. B. Cegesoft^{®} C 24), Hexyldecylstearat (Eutanol^{®} G 16), Hexyldecyllaurat, Isodecylneopentanoat, Isononylisononanoat, 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanot, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat;
- den Benzoesäureestern von linearen oder verzweigten C₈₋₂₂-Alkanolen, z. B. die Handelsprodukte Finsolv^{®} TN (C₁₂-C₁₅-Alkylbenzoat), Finsolv^{®} SB (Isostearylbenzoat) und Finsolv^{®} EB (Ethylhexylbenzoat);
- den C₈-C₂₂-Fettalkoholestern einwertiger oder mehrwertiger C₂-C₇-Hydroxycarbonsäuren, insbesondere den Estern der Glycolsäure, Milchsäure, Äpfelsäure, Weinsäure, Citronensäure und Salicylsäure. Solche Ester auf Basis von linearen C_{12/15}-Alkanolen, z. B. C₁₂-C₁₅-Alkyllactat, und von in 2-Position verzweigten C_{12/13}-Alkanolen, z. B. Di-C₁₂-C₁₃-Alkylmalat, sind unter dem Warenzeichen Cosmacol^{®} von der Firma Nordmann, Rassmann GmbH & Co, Hamburg, zu beziehen, insbesondere die Handelsprodukte Cosmacol^{®} EMI, Cosmacol^{®} ESI und Cosmacol^{®} ETI;
- den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57), PPG-3-Myristylether (Witconol^{®} APM) und PPG-15-Stearylether (Arlamol^{®} E);
- flüssigen Paraffinölen, Isoparaffinölen, z. B. die Handelsprodukte der Permethyl^{®}-Serie, insbesondere Isododecan, Isohexadecan und Isoeicosan, und synthetischen Kohlenwasserstoffen wie Polyisobuten oder Polydecene und alicyclischen Kohlenwasserstoffen, z. B. das Handelsprodukt 1,3-Di-(2-ethyl-hexyl)-cyclohexan (Cetiol^{®} S);
- den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Besonders bevorzugte Alkoholöle sind beispielsweise Hexyldecanol (Eutanol^{®} G), Octyldodecanol und 2-Ethylhexylalkohol;
- Mischungen aus Guerbetalkoholen und Guerbetalkoholestern, z. B. das Handelsprodukt Cetiol^{®} PGL (Hexyldecanol und Hexyldecyllaurat).
- den symmetrischen, unsymmetrischen oder cyclischen Estern der Kohlensäure mit Fettalkoholen, beispielsweise Glycerincarbonat, Dicaprylylcarbonat (Cetiol^{®} CC) oder die Ester der DE-OS 197 56 454;
- Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten;
- Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat;
- Di-n-alkylethern mit insgesamt 12 bis 36, insbesondere 12 bis 24 C-Atomen, z. B. Din-octylether (Cetiol^{®} OE), Di-n- n-Hexyl-n-octylether und n-Octyl-n-decylether.

Besonders bevorzugte Öle sind die flüchtigen cyclischen Siliconöle Decamethylcyclopentasiloxan und Dodecamethylcyclohexasiloxan, die flüchtigen linearen Siliconöle Hexamethyldisiloxan (L₂), Octamethyltrisiloxan (L₃) und Decamethyltetrasiloxan (L₄) sowie beliebige Zweier- und Dreiermischungen aus L₂, L₃ und/oder L₄, flüchtige und nichtflüchtige lineare Siliconöle aus der Serie Dow Corning 200 Fluid mit Viskositäten von 0,65, 1,0, 1,5 und 5 cSt, die Esteröle 2-Ethylhexylpalmitat (z. B. Cegesoft^{®} C 24), Hexyldecyllaurat, 2-Ethylhexylstearat, Isopropylmyristat, Isopropylpalmitat und 2-Ethylhexyllaurat, die Benzoesäureester von linearen oder verzweigten C₈₋₂₂-Alkanolen, insbesondere das Handelsprodukt Finsolv^{®} TN (C₁₂-C₁₅-Alkylbenzoat), C₁₂-C₁₅-Alkyllactat, Di-C₁₂-C₁₃-Alkylmalat, PPG-14-Butylether (Ucon Fluid^{®} AP), die Handelsprodukte der Permethyl^{®}-Serie, insbesondere Isododecan, Isohexadecan und Isoeicosan, sowie Polyisobuten und Polydecene sowie Mischungen der genannten Komponenten.
Es kann erfindungsgemäß bevorzugt sein, Mischungen der vorgenannten Öle einzusetzen. Dabei sind besonders Mischungen aus zwei Ölkomponententypen, z. B. flüchtiges Siliconöl und Esteröl, bevorzugt. Besonders bevorzugt sind Ölmischungen, die mindestens ein flüchtiges cyclisches und/oder lineares Siliconöl enthalten. Außerordentlich bevorzugt sind Ölmischungen, die überwiegend, das heißt zu mehr als 50 Gew.-%, bezogen auf die Ölmischung, mindestens ein flüchtiges cyclisches und/oder lineares Siliconöl enthalten. Weiterhin bevorzugt sind Ölmischungen, die 60 - 95 Gew.-%, besonders bevorzugt 70 - 90 Gew.-%, mindestens eines flüchtigen cyclischen und/oder linearen Siliconöls in Kombination mit 5 - 40 Gew.-%, besonders bevorzugt 10 - 30 Gew.-%, mindestens eines Esteröls, insbesondere eines der vorgenannten Esteröle, enthalten, jeweils bezogen auf das Gewicht der Ölmischung.

In einer weiteren bevorzugten Ausführungsform der Erfindung weist ein Anteil der Ölkomponenten von mindestens 80 Gew.-% einen Brechungsindex n_{D} von 1,39 - 1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen.

Weitere erfindungsgemäß bevorzugte kosmetische Produkte sind dadurch gekennzeichnet, dass 5-50 Gew.-%, bevorzugt 10 - 40 Gew.-%, besonders bevorzugt 12 - 25 Gew.-% der unter Normalbedingungen flüssigen Ölkomponenten ausgewählt sind aus Isopropylmyristat, Isopropylpalmitat, Isohexadecan, Isoeicosan, PPG-14-Butylether, PPG-15-Butylether, 2-Hexyldecanol, Isostearylbenzoat, Dimethicone-PEG/PPG-20/23-benzoat, PPG-53-Butylether, Isostearyllactat, Isostearylpalmitat, Hexyldecyllaurat, Mischungen aus Hexyldecanol und Hexyldecyllaurat, Isocetylpalmitat, 2-Octyldodecanol, Polydecene, Isocetylstearat, 2-Ethylhexylstearat, Hexyldecylstearat, 16-Methyl-1-heptadecanol, Diethylhexylcyclohexan, 2-Ethylhexyllaurat, Benzyllaurat, C₁₂-C₁₅-Alkylbenzoat, Octyldodecylbenzoat, C₁₂-C₁₅-Alkyllactat, Dimethicone PEG-8-benzoat, PPG-5-buteth-7, PPG-2-isodeceth-12, Polyphenylmethylsiloxane, insbesondere Phenyltrimethicone, PPG-2-ceteareth-9, Isostearylisostearat, Di-C₁₂-C₁₃-Alkylmalat, Isododecan, Polyisobuten und Glycereth-7-benzoat sowie Mischungen dieser Komponenten. Außerordentlich bevorzugte Ölkomponenten sind ausgewählt aus C₁₂-C₁₅-Alkylbenzoat (z. B. das Handelsprodukt Finsolv TN), Octyldodecylbenzoat, C₁₂-C₁₅-Alkyllactat, Phenyltrimethicone, Di-C₁₂-C₁₃-Alkylmalat und Polyisobuten.

Die Wasser-in-Öl-Emulsion erfindungsgemäß bevorzugter kosmetischer Produkte umfasst eine Wasserphase, die bevorzugt 40 - 99 Gew.-%, besonders bevorzugt 50 - 90 Gew.-% und außerordentlich bevorzugt 60 - 70 - 85 Gew.-%, jeweils bezogen auf das Gesamtgewicht der treibgasfreien Emulsion, ausmacht. Die Emulgatoren werden erfindungsgemäß weder zur Wasserphase noch zur Ölphase gezählt.
Zur Wasserphase zählen erfindungsgemäß Wasser sowie alle wasserlöslichen Inhaltsstoffe, mit Ausnahme der Emulgatoren.
Bevorzugte kosmetischer Produkte sind durch einen Wassergehalt von 20 - 50 Gew.-%, bevorzugt 25 - 45 Gew.-% und besonders bevorzugt 30 - 40 Gew.-%, jeweils bezogen auf das Gesamtgewicht der treibgasfreien Emulsion, gekennzeichnet.

Erfindungsgemäß bevorzugte wasserlösliche kosmetische oder dermatologische Wirkstoffe sind Antitranspirant-Wirkstoffe. Erfindungsgemäß bevorzugte W/O-Emulsionen enthalten mindestens einen wasserlöslichen Antitranspirant-Wirkstoff. Erfindungsgemäß bevorzugte Antitranspirant-Wirkstoffe sind die wasserlöslichen adstringierenden anorganischen und organischen Salze des Aluminiums, Zirkoniums und Zinks bzw. beliebige Mischungen dieser Salze. Besonders bevorzugte Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Aluminiumundecylenoylkollagenaminosäure, Kaliumaluminiumsulfat (KAl(SO₄)₂. 12 H₂O, Alaun), Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. In einer bevorzugten Ausführungsform enthält die Zusammensetzung ein adstringierendes Aluminiumsalz, insbesondere Aluminiumchlorohydrat, das beispielsweise pulverförmig als Micro Dry^{®} von Reheis, in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders vorteilhaft sein.
Die Antitranspirant-Wirkstoffe können als wässrige Lösungen eingesetzt werden. Bevorzugte erfindungsgemäße kosmetische Produkte sind dadurch gekennzeichnet, dass die W/O-Emulsionen mindestens ein Antitranspirant-Salz in einer Gesamtmenge von 15 - 55 Gew.-%, vorzugsweise 25 - 50 Gew.-% und insbesondere 30 - 40 Gew.-%, jeweils bezogen auf das Gewicht der Aktivsubstanz pro Gewicht der gesamten treibgasfreien W/O-Emulsion, enthalten.

Besonders bevorzugte erfindungsgemäße kosmetische Produkte sind dadurch gekennzeichnet, dass die W/O-Emulsionen mindestens ein Antitranspirant-Salz enthalten und 5 - 50 Gew.-%, bevorzugt 10 - 40 Gew.-%, besonders bevorzugt 12 - 25 Gew.-% der unter Normalbedingungen (20 °C, 1013 mbar) flüssigen Ölkomponenten ausgewählt sind aus Isopropylmyristat, Isopropylpalmitat, Isohexadecan, Isoeicosan, PPG-14-Butylether, PPG-15-Butylether, 2-Hexyldecanol, Isostearylbenzoat, Dimethicone-PEG/PPG-20/23-benzoat, PPG-53-Butylether, Isostearyllactat, Isostearylpalmitat, Hexyldecyllaurat, Zweiermischungen aus Hexyldecanol und Hexyldecyllaurat, Isocetylpalmitat, 2-Octyldodecanol, Polydecene, Isocetylstearat, 2-Ethylhexylpalmitat, 2-Ethylhexylstearat, Hexyldecylstearat, 16-Methyl-1-heptadecanol, Diethylhexylcyclohexan, 2-Ethylhexyllaurat, Benzyllaurat, C₁₂-C₁₅-Alkylbenzoat, Octyldodecylbenzoat, C₁₂-C₁₅-Alkyllactat, Dimethicone PEG-8-benzoat, PPG-5-buteth-7, PPG-2-isodeceth-12, Polyphenylmethylsiloxane, insbesondere Phenyltrimethicone, PPG-2-ceteareth-9, Isostearylisostearat, Di-C₁₂-C₁₃-Alkylmalat, Isododecan, Polyisobuten und Glycereth-7-benzoat sowie Mischungen dieser Komponenten, besonders bevorzugt ausgewählt sind aus C₁₂-C₁₅-Alkylbenzoat, Octyldodecylbenzoat, C₁₂-C₁₅-Alkyllactat, Phenyltrimethicone, Di-C₁₂-C₁₃-Alkylmalat und Polyisobuten. Der Rest der unter Normalbedingungen flüssigen Ölkomponenten ist ausgewählt aus flüchtigen cyclischen Siliconölen, insbesondere Cyclopentasiloxan, Cyclohexasiloxan, Hexamethyldisiloxan, Octamethyltrisiloxan und Decamethyltetrasiloxan sowie Mischungen hiervon. Die genannten Kombinationen aus Antitranspirant-Salzen und den ausgewählten Ölkomponenten, insbesondere Antitranspirant-Salz und 2-Ethylhexylpalmitat und Cyclopentasiloxan und Cyclohexasiloxan oder Antitranspirant-Salz und C₁₂-C₁₅-Alkylbenzoat und Cyclopentasiloxan und Cyclohexasiloxan, führt überraschenderweise dazu, dass die Emulsionen auf der Haut nur geringfügige oder sogar keine sichtbaren Rückstände hinterlassen und aber auch die Kleidung nicht anschmutzen. Weiterhin führen die bevorzugten Ölkomponentenmischungen in Kombination mit den nicht-metallischen Ventilen überraschenderweise zu einem besonders vorteilhaften Sprühbild, das heißt, zu einem Öffnungskegel des Sprühstrahls von 25° bis 65°, bevorzugt 30° bis 60° und besonders bevorzugt 35° bis 55°. Ohne an diese Theorie gebunden sein zu wollen, wird vermutet, dass die bevorzugten Ölkomponenten die rheologischen Eigenschaften der W/O-Emulsionen positiv beeinflussen, so dass es im Kontakt mit den nicht-metallischen Ventilen und Ventilteilen nicht zu störenden Einflüssen kommt.

Weitere erfindungsgemäß bevorzugte kosmetische Wirkstoffe sind Deodorant-Wirkstoffe, die besonders bevorzugt ausgewählt sind aus Geruchsabsorbern, deodorierend wirkenden lonenaustauschern, keimhemmenden Mitteln, präbiotisch wirksamen Komponenten sowie Enzyminhibitoren oder, besonders bevorzugt, Kombinationen der genannten Wirkstoffe.
Silicate dienen als Geruchsabsorber, die gleichzeitig auch die rheologischen Eigenschaften der W/O-Emulsionen der erfindungsgemäßen Produkte vorteilhaft unterstützen können. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Gesamtmenge von 0,1 - 10 Gew.-%, besonders bevorzugt 0,5 - 7 Gew.-% und außerordentlich bevorzugt 1 - 5 Gew.-%, jeweils bezogen auf das Gewicht der W/O-Emulsion, eingesetzt.
Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Arylalkohole wie insbesondere Phenoxyethanol, 2-Methyl-4-phenylbutan-2-ol und 2-Methyl-5-phenylpentan-1-ol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester, insbesondere Carbonsäuremonoester des Mono-, Di- und Triglycerins (insbesondere Glycerinmonolaurat, Diglycerinmonocaprinat, Diglycerinmonolaurat, Triglycerinmonolaurat und Triglycerinmonomyristat), Lantibiotika sowie Pflanzenextrakte (z. B. grüner Tee und Bestandteile des Lindenblütenöls) besonders bevorzugt einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
Weitere bevorzugte Deodorant-Wirkstoffe sind ausgewählt aus den keimhemmend wirkenden Parfümölen und den Deosafe-Parfümölen, die von der Firma Symrise, vormals Haarmann und Reimer, erhältlich sind.
Zu den Enzyminhibitoren gehören Stoffe, die die für die Schweißzersetzung verantwortlichen Enzyme, insbesondere Arylsulfatase, β-Glucuronidase, Aminoacylase, die esterspaltenden Lipasen und die Lipoxigenase, hemmen, z. B. Trialkylcitronensäureester, insbesondere Triethylcitrat, oder Zinkglycinat.
Die Deodorant-Wirkstoffe können sowohl einzeln als auch in Mischungen eingesetzt werden. Besonders bevorzugt sind Phenoxyethanol, α-(2-Ethylhexyl)glycerinether, Diglycerinmonocaprinat, 2-Methyl-5-phenylpentan-1-ol, Mischungen aus Phenoxyethanol und α-(2-Ethylhexyl)glycerinether sowie Mischungen aus Arylalkoholen, insbesondere Phenoxyethanol, mit α-(2-Ethylhexyl)glycerinether und Diglycerinmonocaprinat, insbesondere die Mischungen aus α-(2-Ethylhexyl)glycerinether und 2-Methyl-5-phenylpentan-1-ol.
Die Gesamtmenge der Deodorant-Wirkstoffe in den erfindungsgemäß eingesetzten Emulsionen beträgt bevorzugt 0,1 - 10 Gew.-%, besonders bevorzugt 0,2 - 7 Gew.-%, insbesondere 0,3 - 5 Gew.-% und außerordentlich bevorzugt 0,4 - 1,0 Gew.-%, jeweils bezogen auf das Gesamtgewicht der W/O-Emulsion.

Die Wasser-in-Öl-Emulsion des erfindungsgemäßen kosmetischen Produktes enthält weiterhin mindestens einen Wasser-in-Öl-Emulgator. Der mindestens eine Wasser-in-Öl-Emulgator ist bevorzugt in einer Menge von 0,5 - 5 Gew.-%, besonders bevorzugt 1,0 - 1,5 - 2,5 Gew.-%, jeweils bezogen auf das Gesamtgewicht der Emulsion, enthalten.
Eine erfindungsgemäß besonders bevorzugte Gruppe von Wasser-in-Öl-Emulgatoren sind die Poly-(C₂-C₃)alkylenglycol-modifizierten Silicone, deren frühere INCl-Bezeichnung Dimethicone Copolyol lautete, mit den aktuellen INCl-Bezeichnungen PEG-x Dimethicone (mit x = 2 - 20, bevorzugt 3-17, besonders bevorzugt 11-12), Bis-PEG-y Dimethicone (mit y = 3-25, bevorzugt 4 - 20), PEG/PPG a/b Dimethicone (wobei a und b unabhängig voneinander für Zahlen von 2- 30, bevorzugt 3-30 und besonders bevorzugt 12-20, insbesondere 14-18, stehen), Bis-PEG/PPG-c/d Dimethicone (wobei c und d unabhängig voneinander für Zahlen von 10-25, bevorzugt 14-20 und besonders bevorzugt 14-16, stehen) und Bis-PEG/PPG-e/f PEG/PPG g/h Dimethicone (wobei e, f, g und h unabhängig voneinander für Zahlen von 10-20, bevorzugt 14-18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).
Weitere erfindungsgemäß bevorzugte W/O-Emulgatoren sind Poly-(C₂-C₃)alkylenglycolmodifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich als Abil EM 90 oder in einer Mischung aus Polyglyceryl-4-isostearat, Cetyl PEG/PPG-10/1 Dimethicone und Hexyllaurat unter der Handelsbezeichnung Abil WE 09), weiterhin Alkyl Methicone Copolyole und Alkyl Dimethicone Ethoxy Glucoside.

Weitere erfindungsgemäß geeignete W/O-Emulgatoren sind ausgewählt aus Substanzen der allgemeinen Formel A-O-(CHR¹-X-CHR²-O-)ₐ-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe >CHOR³ darstellt, R¹ und R² ein Wasserstoffatom oder eine Methylgruppe darstellen und so gewählt werden, dass nicht beide Reste gleichzeitig Methyl darstellen, und R³ ein Wasserstoffatom oder eine verzweigte oder unverzweigte, gesättigte oder ungesättigte Alkyl- oder Acylgruppe mit 1 bis 20 Kohlenstoffatomen darstellt.
Besonders bevorzugt ist es, wenn der W/O-Emulgator oder die W/O- Emulgatoren so gewählt werden, dass die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H)_{b}-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.
Weitere bevorzugte W/O-Emulgatoren sind ausgewählt aus
(1) gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden;
(2) ethoxylierten Alkoholen und Carbonsäuren mit 8 - 24 C-Atomen, insbesondere mit 16 - 22 C-Atomen, die einen HLB-Wert von 1 - 8 aufweisen;
(3) propoxylierten Alkoholen und Carbonsäuren mit 8 - 24 C-Atomen, insbesondere mit 16 - 22 C-Atomen;
(4) Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar.
(5) Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Veresterungsgrad von 1-10, vorzugsweise 1-5;
(6) Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1-10, vorzugsweise 1-5;
(7) Propylenglycolestern gesättigter und/oder ungesättigter, verzweigter und/oder unverzweigter Alkancarbonsäuren einer Kettenlänge von 8 - 24, insbesondere 12 - 18 C-Atomen;
(8) Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(9) Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen.
Es kann erfindungsgemäß von Vorteil sein, dass niedrig ethoxylierte (3 - 5 EO) und/oder propoxylierte Produkte Verwendung finden, beispielsweise polyethoxyliertes hydrogeniertes oder nichthydrogeniertes Ricinusöl oder ethoxyliertes Cholesterin.

Besonders bevorzugte W/O-Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Batylalkohol, Polyethylenglycol(2)stearyl-ether (Steareth-2), Glycerylsorbitanstearat, Polyglyceryl-4-Isostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyceryl-3-diisostearat, PEG-8 Distearat, Diglycerin Dipolyhydroxystearat, Glycerinisostearat, Sorbitanisostearat, Polyglyceryl-3-methylglucosedistearat, polyethoxyliertes hydrogeniertes oder nichthydrogeniertes Ricinusöl, ethoxyliertes Cholesterin, PEG-2 Stearat, PEG-45/Dodecylglycolcopolymer, PEG-22/Dodecylglycolcopolymer und Methoxy PEG- 22/Dodecyl Glycol Copolymer.
Ganz besonders bevorzugt ist es, wenn Kombinationen der oben genannten W/O-Emulgatoren, insbesondere eine Kombination aus zwei Emulgatoren, eingesetzt werden.
Es kann erfindungsgemäß vorteilhaft sein, zusätzlich zu dem mindestens einen W/O-Emulgator auch mindestens einen O/W-Emulgator einzusetzen.

Die erfindungsgemäß eingesetzte W/O-Emulsion kann zusätzlich Ethanol enthalten. Ethanol ist zum Beispiel bevorzugt, wenn der Frischeeffekt, der durch den hohen Wassergehalt der erfindungsgemäßen W/O-Emulsionen hervorgerufen wird, weiter gesteigert werden soll.

In einer weiteren bevorzugten Ausführungsform enthalten die erfindungsgemäßen W/O-Emulsionen mindestens ein wasserlösliches Polyol, ausgewählt aus den wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 besonders bevorzugt sind. Auch Zucker und bestimmte Zuckerderivate wie Fructose, Glucose, Maltose, Maltitol, Mannit, Inosit, Sucrose, Trehalose und Xylose können erfindungsgemäß bevorzugt sein. Besonders bevorzugt sind 1,2-Propylenglycol, Glycerin, 1,3-Butylenglycol, Diglycerin, Triglycerin, Dipropylenglycol und Tripropylenglycol.
Die bevorzugten erfindungsgemäßen W/O-Emulsionen enthalten das wasserlösliche mehrwertige C₂ - C₉-Alkanol mit 2 - 6 Hydroxylgruppen und/oder das wasserlösliche Polyethylenglycol mit 3-20 Ethylenoxid-Einheiten bevorzugt insgesamt in Mengen von 0,5 - 25 Gew.-%, besonders bevorzugt 1-20 Gew.-% und außerordentlich bevorzugt 3 - 15 Gew.-% bzw. 5-10 Gew.-%, jeweils bezogen auf die gesamte treibgasfreie W/O-Emulsion.

Die erfindungsgemäß verwendeten W/O-Emulsionen können weiterhin bevorzugt ein oder mehrere Konservierungsmittel enthalten. Erfindungsgemäß bevorzugte Konservierungsmittel sind Formaldehydabspalter (wie z. B. 1,3-Dimethylol-4,4-dimethylhydantoin, INCI-Bezeichnung DMDM Hydantoin, z. B. unter der Handelsbezeichnung Glydant von der Firma Lonza erhältlich), lodopropylbutylcarbamate wie 3-lod-2-propinylbutylcarbamat (z. B. die unter den Handelsbezeichnungen Glycacil-L, Glycacil-S von der Firma Lonza erhältlichen und/oder Dekaben LMB von Jan Dekker), Parabene (d. h. p- Hydroxybenzoesäurealkylester, wie Methyl-, Ethyl-, Propyl- und/oder Butylparaben), Phenoxyethanol, Ethanol, Benzoesäure, Dibromdicyanobutan (2-Brom-2-brommethylglutarodinitril), 2-Brom-2-nitro-propan-1,3-diol, Imidazolidinylharnstoff, 5-Chlor-2-methyl-4-isothiazolin-3-on, 2- Chloracetamid, Benzalkoniumchlorid, Benzylalkohol, Salicylsäure und Salicylate.
Erfindungsgemäß besonders bevorzugte Konservierungsmittel sind ausgewählt aus lodopropylbutylcarbamaten, Parabenen (Methyl-, Ethyl-, Propyl- und/oder Butylparaben) und/oder Phenoxyethanol.

Die Konservierungsmittel sind bevorzugt in Mengen von 0,01 - 2, besonders bevorzugt 0,1 - 1,5 und außerordentlich bevorzugt 0,2 - 1,0 Gew.-% enthalten, jeweils bezogen auf das Gewicht der treibgasfreien W/O-Emulsion.

Die erfindungsgemäß eingesetzten W/O-Emulsionen können bevorzugt weiterhin mindestens eine Duftstoffkomponente enthalten. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Phenoxyethylisobutyrat, Benzylacetat, p-tert.-Butylcyclohexylacetat, Dimethylbenzylcarbinylacetat, Linalylacetat, Phenylethylacetat, Linalylbenzoat, Ethylmethylphenylglycinat, Benzylformiat, Allylcyclohexylpropionat, Styrallylpropionat und Benzylsalicylat. Zu den Ethern zählen zum Beispiel Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die lonone α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Geeignete Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl. Die Duftstoffkomponente/n sind bevorzugt in Gesamtmengen von 0,01 bis 4 Gew.-%, besonders bevorzugt 0,5 - 2 Gew.-%, jeweils bezogen auf das Gewicht der treibgasfreien W/O-Emulsion, enthalten.

Die erfindungsgemäß eingesetzten W/O-Emulsionen können vorteilhafterweise weiterhin mindestens einen hautkühlenden Wirkstoff enthalten. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2- Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.
Die erfindungsgemäß verwendeten W/O-Emulsionen enthalten bevorzugt mindestens einen hautkühlenden Wirkstoff in Mengen von 0,01 - 1 Gew.%, besonders bevorzugt 0,02 - 0,5 Gew.% und außerordentlich bevorzugt 0,05 - 0,2 Gew.%, jeweils bezogen auf das Gesamtgewicht der treibgasfreien W/O-Emulsion.

Die erfindungsgemäß verwendeten W/O-Emulsionen können bevorzugt weiterhin mindestens einen Pflanzenextrakt enthalten. Pflanzenextrakte werden üblicherweise durch Extraktion der gesamten Pflanze, in einzelnen Fällen aber auch ausschließlich aus Blüten und/oder Blättern und/oder Samen und/oder anderen Pflanzenteilen, hergestellt. Erfindungsgemäß bevorzugt sind vor allem die Extrakte aus Aloe Vera, Grünem Tee, Hamamelis, Bambus, Kamille, Ringelblume, Stiefmütterchen, Paeonie, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel und Süßkartoffel. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.
Die erfindungsgemäß verwendeten W/O-Emulsionen können auch Mischungen aus mehreren, insbesondere aus zwei, verschiedenen Pflanzenextrakten enthalten.

Die erfindungsgemäß verwendeten W/O-Emulsionen enthalten mindestens einen Pflanzenextrakt bevorzugt in Mengen von 0,01 - 5 Gew.-%, besonders bevorzugt 0,1 - 2 Gew.-% und außerordentlich bevorzugt 0,5 - 1,0 Gew.-%, jeweils bezogen auf das Gesamtgewicht der treibgasfreien W/O-Emulsion.

In einer weiteren bevorzugten Ausführungsform enthalten die erfindungsgemäß eingesetzten W/O-Emulsionen mindestens ein Vitamin, Provitamin oder eine als Vitaminvorstufe bezeichnete Verbindung aus den Vitamingruppen A, B, C, E, H und K und den Estern der vorgenannten Substanzen.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäß verwendeten W/O-Emulsionen enthalten die Vitamin A-Komponente bevorzugt in Mengen von 0,05 - 1 Gew.-%, bezogen auf die gesamte W/O-Emulsion.
Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte W/O-Emulsion, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte W/O-Emulsion, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte W/O-Emulsion, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

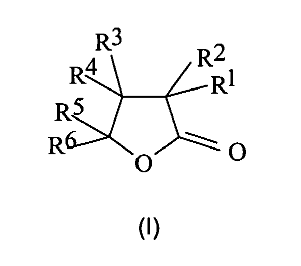

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln bevorzugt in einer Gesamtmenge von 0,05 bis 5 Gew.-%, besonders bevorzugt 0,1 bis 3 Gew.-%, außerordentlich bevorzugt 0,5 bis 2 Gew.-%, jeweils bezogen auf die gesamte treibgasfreie W/O-Emulsion, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen Mitteln bevorzugt in Mengen von 0,0001 bis 1,0 Gew.-%, insbesondere 0,001 bis 0,01 Gew.-%, jeweils bezogen auf die gesamte treibgasfreie W/O-Emulsion, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere 0,001 bis 0,01 Gew.-%, jeweils bezogen auf die gesamte treibgasfreie W/O-Emulsion, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die gesamte treibgasfreie W/O-Emulsion, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die gesamte treibgasfreie W/O-Emulsion, enthalten.

Vitamin C (Ascorbinsäure) wird bevorzugt in Mengen von 0,1 bis 3 Gew.-%, bezogen auf die gesamte treibgasfreie W/O-Emulsion, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte treibgasfreie W/O-Emulsion, enthalten.
Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte treibgasfreie W/O-Emulsion, enthalten.
Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Natriumascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäß verwendeten W/O-Emulsionen mindestens einen hautberuhigenden Wirkstoff. Erfindungsgemäß besonders bevorzugte hautberuhigende Wirkstoffe sind ausgewählt aus Allantoin, α-Bisabolol und α-Liponsäure.
Besonders bevorzugte erfindungsgemäße kosmetische Produkte sind dadurch gekennzeichnet, dass sie mindestens einen hautberuhigenden Wirkstoff in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte treibgasfreie W/O-Emulsion, enthalten.

Kosmetische Zusammensetzungen auf Basis von W/O-Emulsionen, die Antitranspirant-Salze enthalten, können hautaustrocknend wirken. Überraschend wurde festgestellt, dass durch den Zusatz ausgewählter Wirkstoffe ein unerwarteter Ausgleich der negativen Beeinflussung des Hautfeuchtegehaltes und sogar eine hautbefeuchtende Wirkung der erfindungsgemäß verwendeten W/O-Emulsionen erzielt werden konnte. Daher sind weitere erfindungsgemäß bevorzugte kosmetische Produkte dadurch gekennzeichnet, dass die W/O-Emulsion mindestens einen feuchtigkeitsspendenden Wirkstoff, ausgewählt aus Panthenol, Pantolacton, Desoxyzuckern, besonders bevorzugt Rhamnose und Fucose, Polysacchariden, die mindestens einen Desoxyzucker-Baustein enthalten, Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen, enthält. Besonders bevorzugt sind erfindungsgemäße Produkte, die in der W/O-Emulsion mindestens ein Antitranspirant-Salz und mindestens einen feuchtigkeitsspendenden Wirkstoff, ausgewählt aus Panthenol, Pantolacton, Desoxyzuckern, besonders bevorzugt Rhamnose und Fucose, Polysacchariden, die mindestens einen Desoxyzucker-Baustein enthalten, Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen, enthalten.
Besonders bevorzugte erfindungsgemäße kosmetische Produkte sind dadurch gekennzeichnet, dass sie mindestens einen feuchtigkeitsspendenden Wirkstoff, ausgewählt aus Panthenol, Pantolacton, Desoxyzuckern, besonders bevorzugt Rhamnose und Fucose, Polysacchariden, die mindestens einen Desoxyzucker-Baustein enthalten, Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen, in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte treibgasfreie W/O-Emulsion, enthalten.
Außerordentlich bevorzugt sind erfindungsgemäße Produkte mit einer Kombination aus mindestens einem Antitranspirant-Salz auf Basis von Aluminium- und/oder Aluminium/Zirconium-Verbindungen und einem Wirkstoff, ausgewählt aus Panthenol, Pantolacton und N,N'-Bis(2-hydroxyethyl)harnstoff, insbesondere die Kombinationen Aluminiumchlorohydrat und Panthenol, Aluminiumchlorohydrat und Pantolacton, Aluminiumchlorohydrat und N,N'-Bis(2-hydroxyethyl)harnstoff, Aluminium-Zirkonium-Tetrachlorohydrex-Glycin-Komplex und Panthenol, Aluminium-Zirkonium-Tetrachlorohydrex-Glycin-Komplex und Pantolacton, Zirkonium-Tetrachlorohydrex-Glycin-Komplex und N,N'-Bis(2-hydroxyethyl)-harnstoff.

In einer weiteren bevorzugten Ausführungsform enthalten die erfindungsgemäß verwendeten W/O-Emulsionen mindestens einen haarwuchsinhibierenden Wirkstoff. Erfindungsgemäß besonders bevorzugte haarwuchsinhibierende Wirkstoffe sind ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Sérobiologiques mit der INCl-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (pumpkin, Zucchini) und den Früchten von Serenoa serrulata, wie sie beispielsweise und bevorzugt in den Rohstoffen ARP 100 von Greentech S.A./Rahn mit der INCl-Deklaration "Water, Alcohol, Serenoa Serrulata Fruit Extract, Epilobium Angustifolium Extract, Cucurbita Pepo (Pumpkin) Seed Extract" und ARP 100 Huileux (ex Greentech, INCl: Caprylic/Capric Triglyceride, Serenoa Serrulata Fruit Extract, Epilobium Angustifolium Flower/Leaf/Stem Extract, Cucurbita Pepo (Pumpkin) Seed Extract), enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag /Seporga mit der INCl-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind.
Weitere bevorzugte Haarwuchs-inhibierende Wirkstoffe sind ausgewählt aus den Substanzen, die die Protein-Tyrosinkinase hemmen, insbesondere aus Lavendustin-A, Erbstatin, Tyrphostin, Piceatannol, 4-Hydroxybenxylidenmalononitril, 3,5-Di-*tert*-butyl-4-hydroxybenzylidenmalononitril, α-Cyano-(3,4-dihydroxy)-cinnamonitril, α-Cyano-(3,4,5-trihydroxy)cinnamonitril, α-Cyano-(3,4-dihydroxy)cinnamid, α-Cyano-(3,4-dihydroxy)thiocinnamid, 2-Amino-4-(4'-hydroxyphenyl)-1,1,3-tricyanobuta-1,3-dien, 2-Amino-4-(3,4,5'-trihydroxyphenyl)-1,1,3-tricyanobuta-1,3-dien, 2-Amino-4-(1HA-indol-5-yl)-1,1,3-tricyanobuta-1,3-dien, 4-Hydroxy-3-methoxy-5- (benzothiazolylthiomethyl)benzylidencyanoacetamid, 4-Amino-N-(2,5-dihydroxybenzyl)methylbenzoat, α-Cyano-(3,4-dihydroxy)-cinnamonitril, 4-(3-Chloranilino)-6,7-dimethoxychinazolin, α-Cyano-(3,4-dihydroxy)-N-benzylcinnamid, (-)-R-N-(a-Methylbenzyl)-3,4-dihydroxybenzylidencyanoacetamid, α-Cyano-(3,4-dihydroxy)-N-(3- phenylpropyl)-cinnamid, α-Cyano-(3,4-dihydroxy)-N-phenylcinnamid, α-Cyano-(+)-(S)-N-(alpha-phenethyl)-(3,4-dihydroxy)cinnamid,α-Cyano-(3,4-dihydroxy)-N-(phenylbutyl)cinnamid, Herbimycin A, Thiazolidindion, Phenazocin, 2,3-Dihydro-2-thioxo-1H-indol-3-Alkansäuren, 2,2'-Dithiobis-(1H-indol-3-Alkansäuren), ein Sulfonylbenzoylnitrostyrol, Methylcaffeat, HNMPA(AM)₃(hydroxy-2-naphthalenylmethylphosphonsäure-*tris-*acetoxymethylester) und N-Acetyl-Asp-Tyr-(2-malonyl)-Val-Pro-Met-Leu-NH₂.

Bevorzugte erfindungsgemäße Produkte enthalten mindestens eine den Haarwuchs inhibierende Substanz vorzugsweise in einer Menge von 0,1 - 10 Gew.-%, bevorzugt 0,5 - 5 Gew.-% und besonders bevorzugt 1 - 4 Gew.-%, jeweils bezogen auf das Gewicht des Rohstoffs tel quel und das Gesamtgewicht der treibgasfreien W/O-Emulsion.

Weitere besonders bevorzugte erfindungsgemäße Produkte sind dadurch gekennzeichnet, dass die W/O-Emulsion mindestens ein Dimethiconol (S1) enthält. Überraschenderweise wurde festgestellt, dass der Zusatz eines Dimethiconols das Sprühbild des Emulsionssprays verbessert. Hierunter ist bevorzugt zu verstehen, dass das Spray nicht zu stark vernebelt wird, d.h., dass die Spraytröpfchen nicht zu klein sind und in der Luft verbleiben, ohne auf der zu behandelnden Hautoberfläche anzukommen. Weiterhin ist unter einer Verbesserung des Sprühbildes zu verstehen, dass die Emulsion nach dem Aufsprühen auf der Haut verbleibt und nicht wieder "zurückprallt". Weiterhin wurde überraschend festgestellt, dass der Zusatz eines Dimethiconols eine möglicherweise auftretende Verstopfung oder Verklebung der Sprüheinrichtung auch bei längeren Zeitabständen zwischen zwei Sprühstößen deutlich verringert. Die für die W/O-Emulsionen der erfindungsgemäßen Produkte bevorzugten Dimethiconole können linear, verzweigt, cyclisch oder cyclisch mit Verzweigungen sein. Bevorzugte lineare Dimethiconole können durch die folgende Strukturformel (S1 - I) dargestellt werden:

(SiOHR¹₂)-O-(SiR²₂-O-)ₓ- (SiOHR¹₂) (S1 - I)

Bevorzugte verzweigte Dimethiconole können durch die Strukturformel (S1 - II) dargestellt werden:

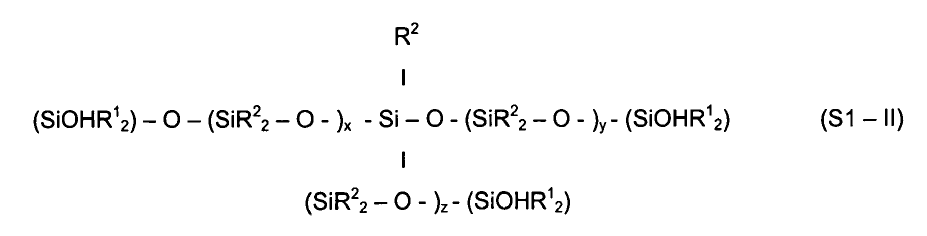

Die Reste R¹ und R² stehen unabhängig voneinander jeweils für Wasserstoff, einen Methylrest, einen C₂ - C₃₀ linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH(CH₃)C(O)OCH₂-, -CH₂CH₂OCH₂-, -OCH₂CH₂-, -C₆H₄C₆H₄-, -OCH₂CH₂CH₂-, -(CH₂)₃CC(O)OCH₂CH₂-, -C₆H₄CH₂C₆H₄-; und -(CH₂)₃C(O)SCH₂CH₂- ein. Bevorzugt als R¹ und R² sind Methyl, Phenyl und C₂ - C₂₂ - Alkylreste. Bei den C₂ - C₂₂-Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Besonders bevorzugt eingesetzte Dimethiconole werden den erfindungsgemäß bevorzugten W/O-Emulsionen nicht in reiner Form, sondern in gelöster Form zugesetzt, beispielsweise als Lösung in einem Cyclomethicone oder einem Dimethicone.
Selbstverständlich umfasst die erfindungsgemäße Lehre auch, dass die Dimethiconole den erfindungsgemäß bevorzugten W/O-Emulsion in bereits voremulgierter Form zugesetzt werden. Dabei kann die entsprechende (Vor-)Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole als Emulsion verwendet werden, dann beträgt die Tröpfchengröße der emulgierten Teilchen erfindungsgemäß 0,01 µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt und bezieht sich auf den Teilchendurchmesser.
Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.
Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemica! Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Erfindungsgemäß besonders bevorzugt ist also ein schweißhemmendes und/oder deodorierendes kosmetisches Produkt, das eine Wasser-in-Öl-Emulsion mit mindestens einem Antitranspirant- und/oder Deodorant-Wirkstoff und mindestens einem Dimethiconol, weiterhin mindestens ein Treibmittel und eine Aerosol-Abgabevorrichtung umfasst, wobei die mit der Emulsion in Kontakt kommenden Teile des Ventils der Abgabevorrichtung aus nicht-metallischen Materialien bestehen und die Emulsion mit einer Sprührate von 0,05 - 0,5 g/s, bevorzugt 0,1 - 0,35 g/s, ausgestoßen wird.

Weitere erfindungsgemäß besonders bevorzugte erfindungsgemäße Produkte sind dadurch gekennzeichnet, dass sie mindestens ein Dimethiconol (S1) in einer Gesamtmenge von 0,001 bis 5 Gew.-%, vorzugsweise 0,01 bis 2 Gew.-%, besonders bevorzugt 0,1 bis 1,0 Gew.-% und insbesondere 0,2 bis 0,5 Gew.-%, bezogen auf das Gewicht der Aktivsubstanz pro Gewicht der treibgasfreien Wasser-in-Öl-Emulsion, enthalten.

Erfindungsgemäß geeignete Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.
Die Menge der Treibmittel beträgt bevorzugt 20 - 80 Gew.-%, besonders bevorzugt 30 - 70 Gew.-% und außerordentlich bevorzugt 40 - 50 Gew.-%, jeweils bezogen auf das Gesamtgewicht der Zubereitung, bestehend aus der W/O-Emulsion und dem Treibmittel.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der Wasser-in-Öl-Emulsion.

Die erfindungsgemäßen kosmetischen Produkte weisen aufgrund der spezifischen Auswahl der Ventilteile trotz der Wasserphase im Aerosolbehälter eine besonders hohe Korrosionsbeständigkeit auf, was einen großen Vorteil gegenüber dem Stand der Technik darstellt. Ferner weisen die erfindungsgemäß eingesetzten Wasser-in-Öl-Emulsionen eine ausgezeichnete Hautverträglichkeit auf. Vorteilhaft ist insbesondere, dass die versprühten Produkte sich auf der Haut durch ein angenehmes, nicht-klebriges Hautgefühl auszeichnen. Der Wassergehalt erzeugt ein deutliches Frischegefühl nach der Anwendung.

Die nachfolgenden Beispiele sollen den Gegenstand der Erfindung erläutern, ohne ihn hierauf zu beschränken.

| Bestandteil | Beispiel 1 | Beispiel 1 | Beispiel 2 | Beispiel 2 |
|---|---|---|---|---|
| | [Gew.-%, bezogen auf Gesamtzubereitung incl. Treibgas] | [Gew.-%, bezogen auf W/O-Emulsion] | [Gew.-%, bezogen auf Gesamtzubereitung incl. Treibgas] | [Gew.-%, bezogen auf W/O-Emulsion] |
| Dow Corning 345 Fluid | 0,48 | 1,6 | 2,173 | 4,1 |
| 1,2-Propylenglycol | 1,5 | 5,0 | 4,823 | 9,1 |
| Dow Corning 5225 C | 4,2 | 12,53 (Cyclomethicone) 1,47(Emulgator) | 7,42 | 12,53 (Cyclomethicone) 1,47(Emulgator) |
| Benzoesäure-C12-15-alkylester | 1,38 | 4,6 | - | - |
| 2-Ethylhexylpalmitat | - | - | 1,219 | 2,3 |
| Parfum | 0,75 | 2,5 | - | - |
| Phenoxyethanol | 0,15 | 0,5 | 0,265 | 0,5 |
| Wasser, vollentsalzt | 1,74 | 5,8 | 2,12 | 4,0 |
| Aluminiumchlorohydrat, 50%ige wässrige Lösung | 19,8 | 66,0 | 34,98 | 66,0 |
| n-Butan | 70,0 | - | 47,0 | - |

Beispielzusammensetzung 1 enthält eine erfindungsgemäße Antitranspirant-Emulsion mit 9,9 Gew.-% Aluminiumchlorohydrat, bezogen auf das Gewicht der treibgasfreien Emulsion. Mit einer Sprührate von 0,35 g/s versprüht man in der Sekunde 0,035 g des schweißhemmenden Wirkstoffs Aluminiumchlorohydrat auf die Hautoberfläche.

Beispielzusammensetzung 2 enthält eine erfindungsgemäße Antitranspirant-Emulsion mit 17,49 Gew.-% Aluminiumchlorohydrat, bezogen auf das Gewicht der treibgasfreien Emulsion. Mit einer Sprührate von 0,2 g/s versprüht man in der Sekunde 0,035 g des schweißhemmenden Wirkstoffs Aluminiumchlorohydrat auf die Hautoberfläche.

### Vergleichsbeispiel:

Ein nicht erfindungsgemäßes Standard-Antitranspirant-Suspensionsspray enthält 5 Gew.-% pulverförmiges Aluminiumchlorhydroxid, bezogen auf das Gewicht der treibgasfreien Suspension. Mit einer Sprührate von 0,7 g/s versprüht man in der Sekunde 0,035 g des schweißhemmenden Wirkstoffs Aluminiumchlorhydroxid auf die Hautoberfläche.

Die Beispiele 1 und 2 zeigen, dass es trotz geringerer Sprührate im Vergleich zu den Standardsprays möglich ist, gleiche Mengen Aluminiumchlorhydroxid bei gleicher Sprühzeit zu applizieren. Die Dosengröße des erfindungsgemäßen Kompaktsprays kann dann um denselben Faktor wie bei der Sprührate kleiner sein, wobei dieselbe Zahl an Anwendungen und dieselbe kosmetische, hier schweißhemmende, Leistung erhalten wird.

Die Beispielzusammensetzungen 1 und 2 wurden in eine Aluminiumdose, die innen mit einem Epoxy-Phenollack beschichtet und mit dem Ventil Ariane M, erhältlich von der Firma Seaquist Perfect, und einem innen mit Microflex-Lack beschichteten Ventilteller ausgerüstet war, abgefüllt und für 12 Wochen bei 45 °C gelagert.
Im Vergleich zu dem gleichen Produkt, das mit einem nicht-erfindungsgemäßen Ventil mit einer Feder, die eine metallische Kontaktfläche mit der W/O-Emulsion aufwies, ausgestattet war, zeigte das erfindungsgemäße Produkt am Ende des Lagertests keine Korrosionsanzeichen an den Ventilteilen. Das nicht-erfindungsgemäße Produkt wies am Ende des 12-wöchigen Lagertests bei 45 °C deutliche Korrosionsanzeichen an der Ventilfeder auf.

### Verwendete Rohstoffe

| | | |
|---|---|---|
| Dow Corning 345 Fluid | Cyclomethicone (Decamethylcydopentasiloxan, Dodecamethylcyclohexasiloxan) | Dow Corning |
| Dow Corning 5225 C Formulation Aid | Cyclomethicone, PEG/PPG-18/18 Dimethicone im Gewichtsverhältnis 8,95:1,05 | Dow Corning |

## Patentansprüche

1. Kosmetisches Produkt, umfassend
- eine Wasser-in-Öl-Emulsion mit mindestens einem kosmetischen oder dermatologischen Wirkstoff,
- mindestens ein Treibmittel und
- eine Aerosol-Abgabevorrichtung, wobei die mit der Emulsion in Kontakt kommenden Teile des Ventils der Abgabevorrichtung aus nicht-metallischen Materialien bestehen,
**dadurch gekennzeichnet, dass** die Emulsion mit einer Sprührate von 0,05 - 0,5 g/s, bevorzugt 0,1 - 0,35 g/s, ausgestoßen wird.

2. Kosmetisches Produkt gemäß Anspruch 1, **dadurch gekennzeichnet, dass** beim Versprühen aus dem Ventil ein Spraystrahl mit einem Öffnungswinkel von mindestens 25° und höchstens 65°, bevorzugt von mindestens 30° und höchstens 60°, ausgestoßen wird.

3. Kosmetisches Produkt gemäß einem der Ansprüche 1 oder 2, **dadurch gekennzeichnet, dass** das Ventil einen Ventilkegel und/oder ein flexibles Element mit Rückstellcharakteristik aufweist, der/das/die mit einem Lack und/oder einem polymeren Kunststoff A beschichtet ist/sind.

4. Kosmetisches Produkt gemäß einem der Ansprüche 1 - 3, **dadurch gekennzeichnet, dass** das flexible Element mit Rückstellcharakteristik als Spiralfeder oder Schraubendruckfeder ausgebildet ist.

5. Kosmetisches Produkt gemäß einem der Ansprüche 1-4, **dadurch gekennzeichnet, dass** das flexible Element mit Rückstellcharakteristik einstückig mit dem Ventilkegel gefertigt ist und biegsame Beine aufweist.

6. Kosmetisches Produkt gemäß einem der Ansprüche 1 - 5, **dadurch gekennzeichnet, dass** das Ventil ein flexibles Element mit Rückstellcharakteristik und/oder einen Ventilkegel aus mindestens einem Kunststoff B aufweist.

7. Kosmetisches Produkt gemäß Anspruch 6, **dadurch gekennzeichnet, dass** der Kunststoff B ein elastomerer Kunststoff ist.

8. Kosmetisches Produkt gemäß einem der Ansprüche 1 oder 2, **dadurch gekennzeichnet, dass** die Abgabevorrichtung ein federloses Ventil aufweist.

9. Kosmetisches Produkt gemäß einem der Ansprüche 1 - 8, **dadurch gekennzeichnet, dass** das Ventil einen innenlackierten Ventilteller aufweist, wobei Lackierung und Ventilmaterial miteinander kompatibel sind.

10. Kosmetisches Produkt gemäß einem der vorstehenden Ansprüche, **dadurch gekennzeichnet, dass** der kosmetische Wirkstoff aus deodorierenden, schweißhemmenden, hautberuhigenden, feuchtigkeitsspendenden und haarwuchsinhibierenden Wirkstoffen, Vitaminen, Provitaminen und als Vitaminvorstufe bezeichneten Verbindungen aus den Vitamingruppen A, B, C, E, H und K und den Estern der vorgenannten Substanzen, und Pflanzenextrakten ausgewählt ist.

11. Kosmetisches Produkt gemäß einem der vorstehenden Ansprüche, **dadurch gekennzeichnet, dass** die Wasser-in-Öl-Emulsion eine Ölphase von 1 - 60 Gew.-%, bezogen auf das Gesamtgewicht der treibgasfreien Emulsion, enthält.

12. Kosmetisches Produkt gemäß einem der vorstehenden Ansprüche, **dadurch gekennzeichnet, dass** die Ölphase zu mindestens 90 Gew.-% aus unter Normalbedingungen flüssigen Ölkomponenten besteht.

13. Kosmetisches Produkt gemäß einem der vorstehenden Ansprüche, **dadurch gekennzeichnet, dass** die Ölkomponenten ausgewählt sind aus Decamethylcyclopentasiloxan, Dodecamethylcyclohexasiloxan, Hexamethyldisiloxan (L₂), Octamethyltrisiloxan (L₃), Decamethyltetrasiloxan (L₄), beliebigen Zweier- und Dreiermischungen aus L₂, L₃ und/ oder L₄, weiterhin 2-Ethylhexylpalmitat, Hexyldecyllaurat, 2-Ethylhexylstearat, 2-Ethylhexyllaurat, Isopropylmyristat, Isopropylpalmitat, C₁₂-C₁₅-Alkylbenzoat, C₁₂-C₁₅-Alkyllactat, Di-C₁₂-C₁₃-Alkylmalat, PPG-14-Butylether, Isododecan, Isohexadecan, Isoeicosan, Polyisobuten und Polydecene sowie Mischungen der genannten Komponenten.

14. Kosmetisches Produkt gemäß einem der vorstehenden Ansprüche, **dadurch gekennzeichnet, dass** die Ölphase eine Ölmischung, die zu mehr als 50 Gew.-% mindestens ein flüchtiges cyclisches oder lineares Siliconöl enthält, umfasst.

15. Kosmetisches Produkt gemäß einem der vorstehenden Ansprüche, **dadurch gekennzeichnet, dass** 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen.

16. Kosmetisches Produkt gemäß einem der vorstehenden Ansprüche, **dadurch gekennzeichnet, dass** mindestens ein Wasser-in-Öl-Emulgator, ausgewählt aus der Gruppe von Poly-(C₂-C₃)alkylenglycol-modifizierten Siliconen mit den INCl-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10-20, bevorzugt 14 - 18 und besonders bevorzugt 16, stehen).

17. Kosmetisches Produkt gemäß einem der vorstehenden Ansprüche, **dadurch gekennzeichnet, dass** die Wasser-in-Öl-Emulsion eine Wasserphase von 40 - 99 Gew.-%, bezogen auf das Gesamtgewicht der treibgasfreien Emulsion, enthält.

18. Kosmetisches Produkt gemäß einem der vorstehenden Ansprüche, **dadurch gekennzeichnet, dass** mindestens ein Antitranspirant-Wirkstoff in einer Menge von 15 - 55 Gew.-%, bezogen auf das Gesamtgewicht der treibgasfreien W/O-Emulsion, enthalten ist.

19. Kosmetisches Produkt gemäß einem der vorstehenden Ansprüche, **dadurch gekennzeichnet, dass** mindestens ein Deodorant-Wirkstoff in einer Menge von 0,1 - 10 Gew.-%, bezogen auf das Gesamtgewicht der treibgasfreien Wasser-in-Öl-Emulsion, enthalten ist.

20. Kosmetisches Produkt gemäß einem der vorstehenden Ansprüche, **dadurch gekennzeichnet, dass** mindestens ein Wasser-in-Öl-Emulgator in einer Menge von 0,5 - 5 Gew.-%, bezogen auf das Gesamtgewicht der treibgasfreien Emulsion, enthalten ist.

21. Kosmetisches Produkt gemäß einem der vorstehenden Ansprüche, **dadurch gekennzeichnet, dass** mindestens ein wasserlösliches mehrwertiges C₂-C₉-Alkanol mit 2 - 6 Hydroxylgruppen und/oder wasserlösliches Polyethylenglycol mit 3-20 Ethylenoxid-Einheiten in einer Menge von 0,5 - 25 Gew.-%, bezogen auf die gesamte treibgasfreie Wasser-in-Öl-Emulsion, enthalten ist.

22. Kosmetisches Produkt gemäß einem der vorstehenden Ansprüche, **dadurch gekennzeichnet, dass** das Treibmittel in einer Menge von 20 - 80 Gew.%, bevorzugt 30 - 70 Gew.% und besonders bevorzugt 40 - 50 Gew.-%, jeweils bezogen auf das Gesamtgewicht der Zubereitung, bestehend aus der W/O-Emulsion und dem Treibmittel, enthalten ist.

23. Kosmetisches Produkt gemäß einem der vorstehenden Ansprüche, **dadurch gekennzeichnet, dass** 5 - 50 Gew.-%, bevorzugt 10 - 40 Gew.-%, besonders bevorzugt 12 - 25 Gew.-% der unter Normalbedingungen flüssigen Ölkomponenten ausgewählt sind aus Isopropylmyristat, Isopropylpalmitat, Isohexadecan, Isoeicosan, PPG-14-Butylether, PPG-15-Butylether, 2-Hexyldecanol, Isostearylbenzoat, Dimethicone-PEG/PPG-20/23-benzoat, PPG-53-Butylether, Isostearyllactat, Isostearylpalmitat, Hexyldecyllaurat, Mischungen aus Hexyldecanol und Hexyldecyllaurat, Isocetylpalmitat, 2-Octyldodecanol, Polydecene, Isocetylstearat, 2-Ethylhexylstearat, Hexyldecylstearat, 16-Methyl-1-heptadecanol, Diethylhexylcyclohexan, 2-Ethylhexyllaurat, Benzyllaurat, C₁₂-C₁₅-Alkylbenzoat, Octyldodecylbenzoat, C₁₂-C₁₅-Alkyllactat, Dimethicone PEG-8-benzoat, PPG-5-buteth-7, PPG-2-isodeceth-12, Polyphenylmethylsiloxane, insbesondere Phenyltrimethicone, PPG-2-ceteareth-9, Isostearylisostearat, Di-C₁₂-C₁₃-Alkylmalat, Isododecan, Polyisobuten und Glycereth-7-benzoat sowie Mischungen dieser Komponenten.

24. Kosmetisches Produkt gemäß einem der vorstehenden Ansprüche, **dadurch gekennzeichnet, dass** mindestens ein Dimethiconol enthalten ist.

25. Kosmetisches Produkt gemäß einem der vorstehenden Ansprüche, **dadurch gekennzeichnet, dass** mindestens ein feuchtigkeitsspendender Wirkstoff, ausgewählt aus Panthenol, Pantolacton, Desoxyzuckern, besonders bevorzugt Rhamnose und Fucose, Polysacchariden, die mindestens einen Desoxyzucker-Baustein enthalten, Harnstoff, N,N'-Bis(2-hydroxyethyl)harnstoff, Betain (Me₃N⁺-CH₂-COO⁻), Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen, enthalten ist.
